Europäisches Patentamt

(19) European Patent Office

Office européen des brevets

(11) Numéro de publication: **0 025 369**

**A1**

(12) **DEMANDE DE BREVET EUROPEEN**

(21) Numéro de dépôt: **80400975.1**

(22) Date de dépôt: **27.06.80**

(51) Int. Cl.³: **C 12 N 13/00**
**C 02 F 1/32, C 12 J 1/00**

(30) Priorité: **29.06.79 FR 7917066**

(43) Date de publication de la demande:
**18.03.81 Bulletin 81/11**

(84) Etats Contractants Désignés:
**BE DE GB IT NL**

(71) Demandeur: **Raymond Joel**
**117 Route de Bretagne**
**F-14760 Bretteville Sur Odon(FR)**

(71) Demandeur: **Lecordier, Pierre**
**Le Val Claire**
**F-14680 Bretteville Sur Laize(FR)**

(72) Inventeur: **Raymond, Joel**
**117, Route de Bretagne**
**F-14760 Bretteville S/Odon(FR)**

(74) Mandataire: **Phélip, Bruno et al,**
**c/o Cabinet HARLE & LECHOPIEZ 21, rue de La**
**Rochefoucauld**
**F-75009 Paris(FR)**

(54) **Procédé pour accélérer l'évolution naturelle d'un produit et applications.**

㊸ Le produit est soumis à un rayonnement électromagnéti-que ayant une fréquence voisine de la fréquence de réso-nance moléculaire ou d'un de ses harmoniques dans une gamme de fréquences comprises entre 3000 et 15000 méga-hertz avec une intensité supérieure à 1 milliwatt par cm². Application à l'accélération d'une fermentation, en particulier pour l'obtention de vinaigre, l'épuration des eaux-vannes ou la production de méthane à partir de fumier.

EP 0 025 369 A1

TITRE MODIFIÉ
voir page de garde

"Procédé pour accélérer l'évolution naturelle d'un produit"

Dans la demande de brevet principal, on a décrit un procédé pour accélérer l'évolution naturelle d'un produit, notamment à usage alimentaire, par emploi d'un rayonnement électromagnétique, procédé dans lequel on détermine d'abord, dans une gamme de rayonnements électromagnétiques de fréquences comprises entre 3000 et 15000 mégahertz (MHz), une fréquence propre de résonance moléculaire, ou un de ses harmoniques, puis on soumet le produit à un rayonnement électromagnétique ayant une fréquence voisine de cette fréquence propre ou de son harmonique, avec une intensité supérieure à 1 milliwatt par $cm^2$.

Il est certain que l'évolution naturelle d'un produit est un phénomène complexe et mal défini, et qu'il est impossible, par exemple, d'écrire les équations de toutes les réactions classiques ou physicochimiques qui peuvent intervenir lors du vieillissement d'un produit tel que le vin ou le whisky.

Il est généralement admis que les réactions qui correspondent à l'évolution naturelle des produits sont sous la dépendance de diverses enzymes, ces corps étant définis commes des catalyseurs organiques colloïdaux solubles dans l'eau.

Dans beaucoup de cas, on admet que les enzymes sont synthétisées par des microorganismes présents dans le milieu qui se transforme. Dans des milieux comme le vin ou le whisky, ces micro-organismes sont en faible quantité, car les conditions ne sont plus favorables à leur développement accéléré au moment où l'évolution se poursuit et, pour cette raison, l'évolution naturelle de tels produits est lente.

2

Dans d'autres transformations naturelles, appelées plus généralement fermentations, les micro-organismes sont, au contraire, en abondance, tout au moins au début de la réaction.

Sachant que les rayonnements électromagnétiques dans les fréquences prévues dans le brevet principal sont fréquemment mortelles pour les organismes supérieurs, il était permis de se demander si l'emploi du procédé dans le cas où l'évolution naturelle consiste en une fermentation n'aboutirait pas, au contraire, à ralentir cette évolution par diminution brutale du nombre de micro-organismes.

Les essais qui ont conduit à la présente demande ont conduit à la conclusion inverse, à savoir que, lorsque l'évolution naturelle du produit consiste en une fermentation, le procédé permet d'obtenir des résultats aussi favorables que dans le cas d'une évolution en présence d'un nombre faible de micro-organismes.

La présente invention consiste donc dans l'application du procédé du brevet principal à l'accélération d'une évolution consistant principalement en une fermentation.

A titre d'exemple, on a traité des échantillons de 500 cc de vin de consommation courante à diverses fréquences entre 5000 MHz et 10000 MHz, avec diverses puissances.

Le vin avait été au préalable ensemencé avec la flore acidifiante spécifique de la transformation en vinaigre.

Les résultats se montrèrent assez différents de ceux obtenus avec du vin non ensemencé.

Avec une fréquence de 5000 MHz on n'obtient pas d'effet appréciable et le vin ensemencé évolue de la

3

même manière que le témoin de vin ensemencé non traité.

A 8200 MHz on observe une légère accélération du processus. Cette accélération est très nette à 8600 MHz, et on observe un maximum à 8800 MHz alors qu'à 9000 MHz l'effet est à peu près le même qu'à 8600 MHz. Pour une fréquence de 1000 MHz il apparait un autre phénomène, à savoir que l'évolution de la fermentation est totalement arrêtée, et on peut supposer que les semences de fermentation qui avaient été introduites dans le vin ont été tuées.

A 8800 MHz pour une fréquence de 1 watt d'énergie réelle irradiée pendant 15 minutes, on observe une formation de mousse abondante alors qu'à 8600 MHz et 9000 MHz on observe une légère formation de mousse, et rien pour la fréquence plus haute ou plus basse.

L'échantillon correspondant a été remis pour analyse à un laboratoire officiel, et les mesures faites ont donné les résultats suivants :

Dénombrement de la flore acidifiante totale :

Echantillon : 250.000 (bacilles à coloration de Gram positif et diplocoques) par millilitre.

Témoin : 2500 (bacilles à coloration de Gram positif) Degré acétique :

Echantillon : 4,32 gCH$_3$COOH %

Témoin      : 3,39 gCH$_3$COOH %.

On a constaté, de façon surprenante et inattendue, que le rayonnement électromagnétique, aux puissances utilisées, loin de raréfier la flore microbienne, a entraîné sa multiplication dans un facteur de 100, et que, par voie de conséquence, le titre en acide acétique a augmenté de 27,5 %.

4

Les essais ont été repris en faisant varier la puissance, à la fréquence de 8800 MHz.

On a trouvé qu'avec une puissance plus forte, soit 2 watt, l'évolution de la fermentation était stoppée, et que la transformation en vinaigre était impossible, les germes ayant été vraisemblablement tués.

Avec une puissance plus faible, soit 0,5 watt, il n'y a pas d'accélération de la fermentation, qui ne se poursuit comme pour un échantillon non traité.

Il est difficile de dire si ces résultats imprévus sont dus à une action sur les micro-organismes eux-mêmes et leur vitesse de reproduction, ou à une modification du milieu qui les environne, modification qui créerait des conditions plus favorables à leur alimentation et à leur prolifération.

Des essais similaires ont donné des résultats analogues avec d'autres produits susceptibles de présenter des fermentations diverses. En particulier, des essais ont été faits sur des échantillons d'eaux-vannes, c'est-à-dire d'eaux résiduaires prélevées dans les égouts d'une ville.

On a constaté là aussi l'existence d'une fréquence optimale vers 8500-9000 MHz, pour une énergie optimale. On observe alors un dégagement intense de gaz formés au moins en partie de méthane.

Il apparait donc que les eaux-vannes, dont le traitement par fermentation exige des temps et des espaces considérables, sont justiciables du procédé selon l'invention, ce qui permet d'obtenir des stations d'épuration efficaces pour des investissements et une surface occupée considérablement réduits par rapport aux installations existantes. Une autre application intéressante de l'invention est la fermentation du fumier avec production intensive de méthane qui constitue une source d'énergie intéressante.

5

## REVENDICATIONS

1. Procédé pour accélérer l'évolution naturelle d'un produit, notamment à usage alimentaire, par emploi d'un rayonnement électromagnétique, dans lequel on détermine d'abord, dans une gamme de rayonnements électromagnétiques de fréquences comprises entre 3000 et 15.000 mégahertz (MHz), une fréquence propre de résonance moléculaire, ou un de ses harmoniques, puis on soumet le produit à un rayonnement électromagnétique ayant une fréquence voisine de cette fréquence propre ou de son harmonique, avec une intensité supérieure à 1 milliwatt par $cm^2$ , caractérisé en ce que l'évolution naturelle qu'on accélère consiste principalement en une fermentation.

2. Procédé selon la revendication 1, caractérisé en ce qu'on ensemence le produit, avant de la soumettre au rayonnement électromagnétique, avec une flore microbienne correspondant à la fermentation désirée.

3. Application du procédé selon l'une des revendications 1 ou 2 à l'obtention de vinaigre ou à l'épuration des eaux-vannes ou au traitement du fumier en vue de la production de méthane.

**0025369**

Numéro de la demande

**EP 80 40 0975**

Office européen
des brevets

**RAPPORT DE RECHERCHE EUROPEENNE**

| Catégorie | Citation du document avec indication, en cas de besoin, des parties pertinentes | Revendication concernée |
|---|---|---|
| | **DOCUMENTS CONSIDERES COMME PERTINENTS** | |
| X | <u>LU - A - 28 701</u> (E.M. HUC) <br> * Revendications 1,2,3,8,9,10,11, 12; page 2, alinéas 4,5; page 3, alinéa 2; page 4, alinéas 2-4; page 5, alinéa 8; page 6, alinéa 7 * <br> -- | 1,2 |
| X | NATURE, vol. 222, 21 juin 1969, pages 1199-2000 <br> S.J. WEBB et al.: "Absorption of Microwaves by Microorganisms" <br> * Page 1199, colonne 2, alinéa 2; page 1200, colonne 1, ali- néas 1,2; page 1200, colonne 2, alinéas 1,3 * <br> -- | 1,2 |
| | <u>FR - A - 2 382 200</u> (RAYMOND J. et al.) <br> * Revendications 1-3 * <br> -- | 1 |
| | <u>FR - A - 2 382 201</u> (RAYMOND J. et al.) <br> * Revendications 1,2 * <br> -- | 1 |
| | CHEMICAL ABSTRACTS, vol. 84, no. 25 1976, page 424, no. 178252f Columbus, Ohio, U.S.A. <br> & SU - A - 506 617 (OTKRYTIYA, IZOBRET., PROM. OBRAZTSY, TOVARNYE ZNAKI 1976, 53(10), 67 <br> * Abrégé * <br> -- | 1,2 |
| | CHEMICAL ABSTRACTS, vol. 80, no. | 1,2 |

**CLASSEMENT DE LA DEMANDE (Int. Cl. 3)**

C 12 N 13/00
C 02 F 1/32
C 12 J 1/00

**DOMAINES TECHNIQUES RECHERCHES (Int. Cl. 3)**

C 12 H 1/16
A 23 P 1/00
C 12 N 13/00
A 23 L 2/24
 3/00
H 05 B 9/06
C 02 F 1/32
C 12 P 5/02

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cite pour d'autres raisons

&: membre de la même famille, document correspondant

Le présent rapport de recherche a été établi pour toutes les revendications

| Lieu de la recherche | Date d'achèvement de la recherche | Examinateur |
|---|---|---|
| La Haye | 03-11-1980 | COUCKE |

OEB Form 1503.1 06.78

**Office européen des brevets**

**RAPPORT DE RECHERCHE EUROPEENNE**

| Catégorie | DOCUMENTS CONSIDERES COMME PERTINENTS Citation du document avec indication, en cas de besoin, des parties pertinentes | Revendica-tion concernée | CLASSEMENT DE LA DEMANDE (Int. Cl. 3) |
|---|---|---|---|
| | 13, 1974, page 152, no. 68322p Columbus, Ohio, U.S.A. M.A.K. HAMID et al.: "Effects of microwaves on green algae" & J. MICROWAVE POWER, 1973, 8(3-4), 267-73 * Abrégé * -- | | |
| | CHEMICAL ABSTRACTS, vol. 78, no. 21, page 43, no. 132247f Columbus, Ohio, U.S.A. S.M. ZUBKOVA et al.: "Effect on cm-range electromagnetic oscilla-tions on the intensity of lumi-nescence of luminous bacteria" & TR. MOSK. OBSHCHEST. ISPYT. PRIR. 1972, 39, 116-20 * Abrégé * -- | 1,2 | **DOMAINES TECHNIQUES RECHERCHES (Int. Cl. 3)** |
| | CHEMICAL ABSTRACTS, vol. 69, no. 13, page 4670, no. 50036m Columbus, Ohio, U.S.A. V.P. KOMOV et al.: "Effect of milli-meter-range radiation on the mor-phologic and biochemical features of Cl(ostridium) sporogenes" & TR. LENINGRAD. KHIM.-FARM. INST. 1967, 20(1), 91-8 * Abrégé * -- | 1 | |
| | TABLES DES COMPTES RENDUS DES SE-ANCES DE L'ACADEMIE DES SCIENCES, vol. 281, deuxième semestre 1975, Série D, 22 septembre 1975, pages 843-846 ./. | 1,2 | |

Office européen
des brevets

**RAPPORT DE RECHERCHE EUROPEENNE**

| | | | |
|---|---|---|---|
| **DOCUMENTS CONSIDERES COMME PERTINENTS** | | | **CLASSEMENT DE LA DEMANDE (Int. Cl. 3)** |
| Catégorie | Citation du document avec indication, en cas de besoin, des parties pertinentes | Revendica-tion concernée | |
| | M. ANDRE-JEAN BERTEAUD et al.: "Action d'un rayonnement électromagnétique à longueur d'onde millimétrique sur la croissance bactérienne"<br><br>  * Page 844, figure 2; page 845, alinéas 1-3 *<br><br>-- | | |
| | PHYSICS LETTERS, vol. 62A, no. 6, 19 septembre 1977, pages 463-466 NL.<br>W. GRUNDLER et al.: "Resonant growth rate response of yeast cells irradiated by weak microwaves"<br><br>  * Page 463, colonne 2, alinéas 2,3; tableau 1 *<br><br>-- | 1,2 | **DOMAINES TECHNIQUES RECHERCHES (Int. Cl. 3)** |
| | FR - A - 2 196 295 (J. KREUTER)<br><br>  * Page 2, alinéa 3; page 3, alinéa 4; page 4, alinéas 3,4; page 5, alinéas 1,2; revendications 1,35-37 *<br><br>-- | 1,3 | |
| A | FR - A - 941 382 (J.H. CHEVRIER)<br><br>  * Page 1, lignes 30-60; page 2, lignes 37-43 *<br><br>-- | 1,2 | |
| | FR - A - 754 025 (P. LIEBESNY et al.)<br><br>  * Résumé a,d,e,f; page 2, lignes 26-62; exemple 7 *<br><br>---- | 1-3 | |

OEB Form 1503.2  06.78